# EUROPEAN PATENT SPECIFICATION

(11) **EP 2 919 803 B1**
(45) Date of publication and mention of the grant of the patent: **08.05.2019**
(21) Application number: 13791813.2
(22) Date of filing: 15.11.2013
(51) Int. Cl.: A61K 38/24, A01K 67/02

(54) **SINGLE DOSE RECOMBINANT BOVINE FSH AFTER FOLLICULAR SYNCHRONISATION**
EINZELDOSIS VON REKOMBINANTEM BOVINEN FSH NACH FOLLIKULÄRER SYNCHRONISIERUNG
DOSE UNIQUE DE FSH BOVIN APRES SYNCHRONISATION FOLLICULAIRE

(30) Priority: 16.11.2012 EP 12306428
(43) Date of publication of application: 23.09.2015
(60) Divisional application: 19151721.8
(73) Proprietor: Ceva Santé Animale, 33500 Libourne Cedex (FR)
(72) Inventor: SOUZA, Alexandre, F-33360 Carignan de Bordeaux (FR); ISAKA, Naomi, F-33500 Libourne (FR); CARRIE, Aude, F-33500 Les Billaux (FR); THIBAUD, Dominique, F-33200 Bordeaux (FR)
(74) Representative: Cabinet Becker et Associés
(86) International application number: PCT/EP2013/073928
(87) International publication number: WO 2014/076231

(56) References cited:
- WO-A1-2009/073574
- WO-A2-2008/098169
- Anonymous: "The Estrous Cycle in Cattle - Physiology and Endocrinology", , 18 March 2011 (2011-03-18), page 1, no number, 2-25, 37-43, 53, 55, 56, 28, 60-62,, XP055047804, Retrieved from the Internet: URL:http://extension.missouri.edu/adair/do cuments/Livestock/TheEstrousCycleCattle.pd f [retrieved on 2012-12-13]
- R. L. Larson ET AL: "The Bovine Estrous Cycle and Synchronization of Estrus", , 10 October 2006 (2006-10-10), pages 1-6, XP055047806, Retrieved from the Internet: URL:http://www.vet.k-state.edu/studentorgs /bovine/pdf/Estrous_Cycle_physiology1.pdf [retrieved on 2012-12-13]
- H. TWAGIRAMUNGU ET AL: "Synchronization of ovarian follicular waves with a gonadotropin-releasing hormone agonist to increase the precision of estrus in cattle: a review", JOURNAL OF ANIMAL SCIENCE, vol. 73, no. 10, 1 October 1995 (1995-10-01), pages 3141-3151, XP055047808,
- BERFELT D R ET AL: "Ovarian synchronization following ultrasound-guided transvaginal follicle ablation in heifers", THERIOGENOLOGY, LOS ALTOS, CA, US, vol. 42, no. 6, 1 November 1994 (1994-11-01), pages 895-907, XP023188624, ISSN: 0093-691X, DOI: 10.1016/0093-691X(94)90113-W [retrieved on 1994-11-01]
- NOGUEIRA ET AL: "The effect of type of vaginal insert and dose of pLH on embryo production, following fixed-time AI in a progestin-based superstimulatory protocol in Nelore cattle", THERIOGENOLOGY, LOS ALTOS, CA, US, vol. 67, no. 3, 3 January 2007 (2007-01-03), pages 655-660, XP005819541, ISSN: 0093-691X, DOI: 10.1016/J.THERIOGENOLOGY.2006.10.001
- BARROS CIRO M ET AL: "Use of knowledge regarding LH receptors to improve superstimulatory treatments in cattle", REPRODUCTION FERTILITY AND DEVELOPMENT, C S I R O PUBLISHING, AU, vol. 22, no. 1, 1 January 2010 (2010-01-01), pages 132-137, XP009165737, ISSN: 1031-3613, DOI: 10.1071/RD09227
- BO G A ET AL: "The timing of ovulation and insemination schedules in superstimulated cattle", THERIOGENOLOGY, LOS ALTOS, CA, US, vol. 65, no. 1, 7 January 2006 (2006-01-07), pages 89-101, XP027930015, ISSN: 0093-691X [retrieved on 2006-01-07]
- Gabriel A. Bo: "Recent advances in the control of follicular development and superovulation protocols in cattle", 28th Meeting A.E.T.E., 8 September 2012 (2012-09-08), pages 57-68, XP055093789, Retrieved from the Internet: URL:http://www.aete.eu/pdf_publication/31. pdf#page=71 [retrieved on 2013-12-17]
- ANDRÉS TRÍBULO ET AL: "Superstimulation of ovarian follicular development in beef cattle with a single intramuscular injection of Folltropin-V", ANIMAL REPRODUCTION SCIENCE, vol. 129, no. 1-2, 1 November 2011 (2011-11-01), pages 7-13, XP055047813, ISSN: 0378-4320, DOI: 10.1016/j.anireprosci.2011.10.013
- E. P Lemke ET AL: "Single-chain human gonadotropin analogs induce follicle development in sheep", Journal of Endocrinology, vol. 196, no. 3, 29 February 2008 (2008-02-29), pages 593-600, XP055409301, GB ISSN: 0022-0795, DOI: 10.1677/JOE-07-0473
- Reuben J. Mapletoft ET AL: "Recent advances in the superovulation in cattle", Reproduction Nutrition Development, vol. 42, no. 6, 1 November 2002 (2002-11-01), pages 601-611, XP055409302, ISSN: 0926-5287, DOI: 10.1051/rnd:2002046
- K L Macmillan ET AL: "Effects of an Agonist of Gonadotropin-Releasing Hormone on Ovarian Follicles in Cattle", Biology of Reproduction, vol. 45, 1 January 1991 (1991-01-01), pages 883-889, XP055409309,
- J R Pursley ET AL: "SYNCHRONIZATION OF OVULATION IN DAIRY COWS USING PGF2, AND GnRH", THERIOGENOLOGY, vol. 44, no. 7, 1 November 1995 (1995-11-01), pages 915-923, XP055224659, US ISSN: 0093-691X, DOI: 10.1016/0093-691X(95)00279-H
- Gabriel A. Bo ET AL: "New approaches to superovulation in the cow", Reproduction, Fertility and Development, vol. 22, no. 1, 1 January 2010 (2010-01-01), pages 106-112, XP055409318,

## Description

The present invention relates to a use of a bovine recombinant FSH (rFSH) analog or a composition comprising a bovine recombinant rFSH analog to increase reproductive performance in a bovine, wherein said bovine recombinant rFSH analog comprises a specific amino acid sequence and said rFSH analog is administered to said bovine after follicle synchronization, in one single administration at a dose of 50 µg.

### BACKGROUND OF THE INVENTION

The ability to increase reproductive performance in non-human mammals such as cattle, horses or other ungulates can have a significant benefit to owners. This can be achieved through increasing fertility and/or fecundity in such non-human mammals. Currently, several methods or protocols are proposed to increase reproductive performance, based on the use of estradiol and other hormones such as GnRH (Gonadotropin Releasing Hormone), LH (Luteinizing Hormone), FSH (Follicle stimulating Hormone), or progestagens (such as progesterone) to mimic natural or close to natural levels of hormones occurring at the target specie. Some of these protocols include a step to synchronize ovulation in females to facilitate stimulation, growth and ovulation of follicles in a synchronized fashion allowing the fixed-time artificial insemination, avoiding the necessity of estrus detection. These so called synchronization protocols are widely used in commercial dairy and beef herds worldwide.

One of the most common treatments used to synchronize the emergence of follicular waves involves the use of different types of estrogens. However, this steroid hormone cannot be used in many parts of the world and alternative methods have been developed to control follicular and luteal phases aiming for instance the improvement of superovulation process of embryo-donor cows in countries where estradiol is not available. An approach that seems to be a promising option is to initiate FSH treatments at the time of the emergence of a new follicular wave, following a GnRH-induced ovulation. In addition, because the half-life of currently available FSH products are generally short (approximately 6h) superovulatory protocols involve twice a day FSH treatments throughout 4 to 5 days, which are fairly time consuming, stressful and subject to error. Furthermore, these frequent managements during currently developed superovulation schemes make it barely impossible its application for example in beef cattle kept in pastures. Therefore, the standard superovulatory approach is based on multiple injections of FSH at the time of emergence of follicular waves that normally happens 8 to 12 days after naturally occurring estruses. This method is known as "estrus-based", and while efficient in terms of embryo production, is conditioned by the stage of the estrus cycle and requires estrus detection. Later studies have found that fertilization rates are significantly lower in "estrus-based" programs as compared to modern synchronization protocols that allow a more ideal interval from insemination to ovulation.

Studies have been conducted in order to determine whether lengthening the FSH stimulation protocol could increase the ovulatory response. The authors appear to conclude that extending the FSH injection period from 4 consecutive days to 7 consecutive days could sustain follicle growth and result in more follicles acquiring the capacity to ovulate. More studies are ongoing to confirm this hypothesis in different cattle breeds.

In another reported protocol, cows were treated with FSH during 3 days instead of 4, and the last 2 injections, on day 4, were replaced by 2 eCG (equine Chorionic Gonadotropin) injections (Barros et al., 2008). This treatment appeared to increase the number of embryos, although the time of treatment is still long. In addition, eCG is a fairly long molecule and have been described to induce antibody formation when repeatedly used, and perhaps limiting the wide/frequent use of these types of protocols.

In the attempt to reduce the number of FSH injections, an alternative protocol has been developed wherein FSH is embedded in a slow release polymer (hyaluronan). Using this slow release formulation, a single injection was made after "estrus-based" superovulation schedules. However, the authors conclude that the hyaluronan is either too viscous and that the single intramuscular injection resulted in a lower superovulatory response (Bo et al., 2010).

EP2134165 proposes an alternative method for increasing reproduction using a single-chain FSH wherein both subunits are linked covalently by a peptide linker, which is administered in a single dose 7 to 13 days after estrus. According to this method, the animals are prepared for the FSH injection by identifying the reference estrus (or heat) date (day of last heat) and then the FSH is administered in a single injection between days 7 to 8 of the animal's cycle. The results appear satisfactory, but an individual observation and monitoring of the animals is required to determine the most effective treatment regimen.

### SUMMARY OF THE INVENTION

The present invention relates to a use of a bovine recombinant FSH (rFSH) analog or a composition comprising a bovine recombinant rFSH analog to increase reproductive performance in a bovine, wherein said bovine recombinant rFSH analog comprises an amino acid sequence represented by SEQ ID NO 3 and said rFSH analog is administered to said bovine after follicle synchronization, in one single administration at a dose of 50 µg.

The preferred embodiments are defined in dependent claims 2-12.

The specification also discloses improved methods for increasing reproduction, particularly fertility and fecundity, in bovines.

An object of the invention relates to the use of a recombinant rFSH analog, as defined herein, or of a composition comprising said rFSH analog, for increasing reproductive performance in bovines having undergone previously a follicular synchronization protocol and/or treatment, wherein the said rFSH analog is administered in one single administration at a dose of 50 µg.

Another object of the invention resides in a use of a rFSH analog as defined herein (or a composition comprising said rFSH analog) for increasing reproductive performance in one or several bovines, wherein said rFSH analog is administered to each of said one or several bovines after follicle synchronization, in one single administration at a dose of 50 µg.

Such use is particularly advantageous for improving the performance of *in vitro* fertilization (IVF) protocols or procedures.

In this respect, the specification discloses an *in vitro* fertilization (IVF) method comprising a step of obtaining oocytes from a bovine donor and a step of fertilizing *in vitro* said oocytes, wherein the bovine donor is treated by one single administration of a rFSH analog, as defined herein, at a dose of 50µg, to increase oocyte production and wherein administration is performed after follicle synchronization of the bovine donor.

The specification also discloses a method for improving the quality and quantity of *Corpora Lutea* in a bovine, comprising administering to said bovine a rFSH analog, as defined herein, wherein said rFSH analog is administered to said bovine in one single administration at a dose of 50µg, after follicle synchronization of the bovine.

In a particular embodiment, the invention relates to the use as defined above, comprising:
(a) providing a bovine, or a group of bovines, which has been treated to synchronize follicles; or treating a bovine, or a group of bovines, to synchronize follicle;
(b) administering to said bovine or group of bovines one single dose of said rFSH analog at a dose 50 µg, preferably by injection, more preferably by intramuscular injection; and
(c) optionally inseminating the bovine or group of bovines, preferably by artificial insemination, or collecting oocytes from the bovine or group of bovines, preferably near the time of ovulation and, more preferably, 2 to 7 days, even more preferably 4 to 6 days after administration step (b).

This use avoids the need to detect or monitor estrus, and is more effective especially for groups of bovines.

In another particular embodiment, the invention relates to the use comprising:
(a) treating a bovine or a group of bovines to synchronize follicles;
(b) administering to said bovine or group of bovines one single dose of said rFSH analog at a dose of 50µg, preferably by injection, more preferably by intramuscular injection;
(c) recovering oocytes from the bovine, preferably by follicular aspiration;
(d) optionally, in vitro fertilizing the recovered oocytes; and
(e) optionally implanting the fertilized oocytes in a recipient bovine, preferably in a recipient bovine previously treated by administration of one single dose of said rFSH analog at a dose of 50µg.

The rFSH analog for use in the present invention is a single chain rFSH. The rFSH analog is preferably used in essentially pure form, optionally in association with one or several pharmaceutically acceptable excipients or carriers. The rFSH comprises an amino acid sequence represented by SEQ ID NO. 3.

The use of the invention allows increasing fertility and/or fecundity and, especially, superovulation, ovulation rate, oocyte production, or *Corpora Lutea* (CL) quality and quantity in bovine.

### LEGEND TO THE FIGURES

Figure 1: Protocol used to increase reproductive performance with the rFSH analog.
Figure 2: Nucleotide and amino acid sequences of a bovine rFSH analog, the rFSH analog used in the invention comprising SEQ ID NO. 3. The CTP linker sequence which links the beta and alpha subunits is underlined. The underlined and bold nucleotides encode the signal peptide.

### DETAILED DESCRIPTION OF THE INVENTION

The present specification provides methods to increase reproductive performance in bovines, and preferably cattle. In particular, the recombinant single chain FSH used in the present invention is used to stimulate fertility and/or fecundity in bovine females, as illustrated by a superovulation, an increase of embryo production, and/or an increase of pregnancy. The recombinant single chain FSH as used in the invention is also effective to stimulate the growth and the maturation of follicles, resulting in an improved oocyte recovery, superovulation, and ovulation rate, and in an improved quality and quantity of *Corpora Lutea* (CL).

The recombinant single chain FSH as used in the invention can be used in insemination protocols and/or treatments, including for embryo transplantation and in vitro fertilization of bovine.

### Definitions

Within the context of the present invention, the term "increasing reproductive performance" or "increased reproductive performance" refers to increasing the likelihood that a bovine, or a plurality of bovines, will be fertile and fecund.
Increasing reproductive performance includes a stimulation of the growth and/or maturation of follicles, or an improvement in the quality and quantity of *Corpora Lutea.* Increased reproductive performance also includes an increased likelihood that a bovine, or a plurality of bovines, which has been inseminated will become pregnant, will deliver a live offspring, or develop viable embryos.
Increasing reproductive performance also includes increasing the number of viable embryos of a bovine or a plurality of bovines produce *in utero* and/or *in vitro.* Increased reproductive performance includes increasing fertility, fecundity, superovulation, oocyte rate, ovulation rate, embryo production and/or pregnancies. An increase is preferably by approximately at least 1% as compared to non-treated bovines, more preferably by at least 2%, 3%, 4%, 5%, 10% or more.

The term "fertility" or "fertile" refers, within the context of this invention, to the ability to produce fertilizable oocytes.

The term "fecund" or "fecundity" refers, within the context of this invention, to the ability to complete a pregnancy.

The term "superovulation" refers, within the context of this invention, to an increase in the number of ovulated follicles and/or in the creation of fertile ova.

The term "pregnant" refers to a bovine or to a group of bovines some of which being currently pregnant or that has been inseminated and may be pregnant.

As used herein, the term "estrus" refers to the period during which a bovine is most likely to become pregnant. Estrus may be detected or monitored by behavioral demonstration that a bovine is in heat, including showing standing heat.

"Insemination" refers to introducing semen by any method known in the art, including, but not limited to, natural and Artificial Insemination (AI) and *in vitro* fertilization (IVF).

A "group" of bovines designates any group of at least 2 bovines, such as a herd or flock.

The term "administration" refers to all route of administration such as oral, enteral mucosal, parenteral or percutaneous. Preferably the administration route is an injection.

The term "follicle synchronization" refers, within the context of this invention, to synchronization of the emergence of follicular waves.

### Recombinant FSH analog

Follicle Stimulating Hormone belongs to the class of fertility hormones. FSH is composed of two chains (or subunits), termed alpha and beta. Under physiological conditions, both subunits are linked by non-covalent interaction. The nucleotide and amino acid sequences of FSH from distinct species have been disclosed and are available in public databases such as bovine FSH (Kim KE et al., 1988, "nucleotide sequence of the bovine gene for follicle-stimulating hormone beta-subunit", DNA1988, 7(4): 227-233) or human FSH: gene bank number: CAA43996.1.

The present invention utilizes a FSH analog. The term "analog" designates, generally, a compound which mimics the physiological effect of a natural compound. An analogs is structurally similar to the natural compound, and may present structural differences as a result of, e.g., production methods or because the differences confer a beneficial activity to the analog.

The FSH analog used in the invention is a FSH having both alpha and beta subunits covalently linked. Such FSH analog may also be designated "single chain" FSH.

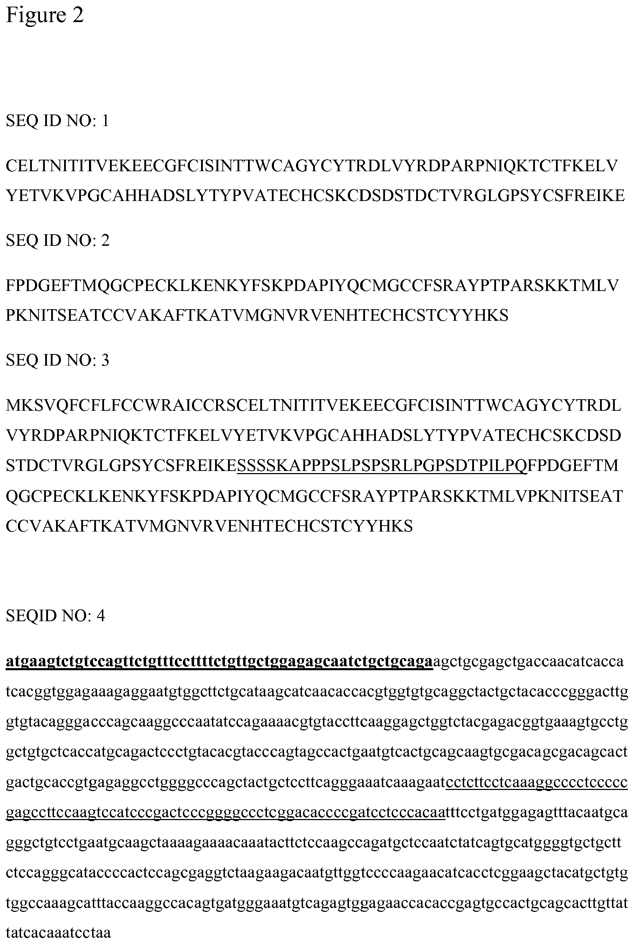

The invention uses a recombinant FSH in which the alpha subunit is covalently linked to the beta subunit by a peptide linker. The invention shows such recombinant single chain analog of FSH provides improved properties for increasing reproductive performance in bovines. The recombinant bovine FSH analog used in the present invention comprises the amino acid sequence as provided in Figure 2: SEQ ID NO: 3. The cDNA corresponds to SEQ ID NO: 4. The first part (amino acids 1-129) corresponds to the sequence of bovine FSH beta subunit (SEQ ID NO 2), the central part corresponds to the carboxy terminal peptide linker (amino acids 130-157), and the third part (amino acids 158-253) corresponds to the sequence of bovine FSH alpha subunit (SEQ ID NO 1, see also, EP2134165).

One embodiment of the invention uses a single chain recombinant bovine FSH having the amino acid sequence of SEQ ID NO 3.

The bovine FSH analog as disclosed above comprises a recombinant FSH wherein the alpha and beta domains derive from bovine FSH and are linked by a peptide linker.

The protein is preferably essentially pure, i.e., with a purity level of at least 95%, more preferably at least 97, 98 or 99%.

In a preferred embodiment, the recombinant single chain FSH as used in the invention is administered in a composition comprising a suitable pharmaceutical formulation. The pharmaceutical formulation may comprise one or several excipients or carriers
In one preferred embodiment, the bovine recombinant single chain FSH as used in the invention has an activity above 10 000IU/mg; more preferably above 13 000IU/mg, such as of about 16 000IU/mg. According to the conversion factor published by the ASRM practice committee (Fertility and Sterility, vol90, supl3, November 2008-American Society for Reproductive Medecine), 10 000IU of bovine FSH correspond to 600µg.

### Treatment

As previously indicated, the invention relates to a use of a recombinant FSH analog as described above for increasing reproductive performance in bovines. The invention may be used in insemination programs particularly bovines artificial insemination, e.g., to improve ovulation rate and/or superovulation and/or the quality and quantity of *Corpora Lutea* in the treated bovines; as well as *in vitro* fertilization programs, e.g., to improve oocyte recovery from donor bovines and/or pregnancy rate in recipients bovines.

More particularly, an object of the invention relates to a use of recombinant FSH analog as defined above to increase reproductive performance in bovines, wherein said recombinant FSH is administered to said bovines after follicle synchronization in one single administration at a dose 50µg.

In a preferred embodiment, the use comprises:
(a) treating a bovine or a group of bovines to synchronize follicles, or providing a bovine or a group of bovines which has been treated to synchronize follicles;
(b) administering to the treated bovine(s) 50µg of the said recombinant FSH analog in one single dose; and
(c) optionally, inseminating the bovine(s) and/or collecting oocytes preferably near the time of ovulation and, more preferably, 2 to 7 days, even more preferably 4 to 6 days after administration step (b).

In a first treatment step, emergence of a new follicular wave is synchronized. The invention shows that, in combination with a recombinant FSH of the invention, such a treatment allows improved embryo production, even at lower dose of hormone and with fewer injections.

Follicular growth is not continuous in bovines, but occurs in waves (2 to 4 waves per cycle). Each wave begins approximately when the dominant follicle in the previous wave gains maximal size, at which time numerous small follicles begin a period of rapid growth. From this group of follicles, one follicle is allowed to grow to a much larger size than the others. This large follicle is called the dominant follicle, because it has the ability to regulate and restrict the growth of the smaller follicles, called subordinate follicles. A few days after reaching maximum size, the dominant follicle begins to regress and die. As the dominant follicle degenerates, its ability to restrict the other follicles is reduced; therefore, a new follicular wave is initiated. A consequence of this dynamic process is that follicles of all sizes, including at least one large follicle, exist on each day of the estrous cycle.

Follicle wave synchronization gives the opportunity to treat all bovines in a limited period of time and, therefore, to capture the economic benefits of the insemination. Upon synchronization of the estrous cycle, a high percentage of treated females show a fertile, closely synchronized estrus and ovulation.

The synchronization of ovulation or timed-AI protocols refers to methods and/or protocols that artificially stimulate follicle growth and timed ovulation, so that ovulation is initiated at a predetermined time with no need to monitor estrus behavior. A review of common methods and protocol applied in bovine are described in the article of Bo et al in the 28th Annual meeting AETE- Saint Malo, France, 7-8th September 2012 (Recent advances in the control of follicular development and superovulation protocols in cattle).

A new follicle wave emergence can be performed by hormonal or physical treatment. Hormonal treatment comprises the administration of suitable hormones such as prostaglandin(s), progestagen(s), or GnRH. Physical treatment includes follicle ablation.

In a preferred embodiment, the follicle synchronization is obtained by hormonal treatment of the bovine(s), preferably with progestagens, progestagen-prostaglandin combinations, prostaglandins alone, progestagen-estrogen combinations, and gonadotropin-prostaglandin combinations with or without progestagens.

In a preferred embodiment, follicle synchronization is obtained by one of the following treatments:
- PGF2alpha or its analogs;
- GnRH + PGF2alpha
- Progestagen (as progesterone...), optionally in combination with estrogen or PGF2alpha or GnRH.

Specific dosages and/or protocols are disclosed in the art such as, e.g., in Thatcher et al., 2001 (American Association of Bovine Practitioner, AABP, Vancouver, 95-105); Diskin et al., 2001 (occasional publication n° 26, p175, British society of Animal Science); or Pursley et al., 1995 (Theriogenology 44 p915). Also, progestagens may be administered using specific devices such as implants (e.g., PRID, of Ceva Santé Animale).

In another embodiment, follicle synchronization is obtained by follicular ablation. Ablation of follicle designates the elimination, removal or destruction of at least one follicle. Follicular ablation refers to physical methods of follicular ablation, such as cautery or ultrasound-guided transvaginal follicle aspiration at random stages of the estrous cycle (Bergfelt, 1997). Ablation can be directed to all follicles or only to the one or two largest follicles (Baracaldo et al., 2000), to ensure at least the dominant follicle is suppressed.

In a preferred embodiment, the invention relates to a use comprising:
(a) treating a bovine or a group of bovines with a progestagen, a progestagen-prostaglandin combination, a prostaglandin, a progestagen-estrogen combination, or a gonadotropin-prostaglandin combination with or without progestagens, to synchronize follicles; or providing a bovine or a group of bovines which has been treated with a progestagen, a progestagen-prostaglandin combination, a prostaglandin, a progestagen-estrogen combination, or a gonadotropin-prostaglandin combination with or without progestagens, to synchronize follicles;
(b) administering to the treated bovines 50µg of the said recombinant FSH analog in one single dose; and
(c) optionally, inseminating a treated bovine of (b) and/or collecting oocytes from a treated bovine of (b), preferably near the time of ovulation and, more preferably, 2 to 7 days, even more preferably 4 to 6 days after administration step (b).

In step (b), the recombinant FSH analog can be administered using any means or techniques known per se in the art including, without limitation, systemic administration, such as intramuscular, intravenous, subcutaneous, etc. Preferred administration route is intramuscular injection.

The results presented in the experimental section show that, on synchronized bovines, such a single administration causes production of fertile embryos and increases substantially the reproductive activity.

In a preferred embodiment, the use further comprises a step (c) of inseminating said ungulate, preferably near the time of ovulation and, more preferably, 2 to 7 days, even more preferably 4 to 6 days after rFSH analog administration.

Additional hormones, such as luteinizing hormone, chorionic gonadotropin and prostaglandin, are optionally administered as well as the rFSH. In one embodiment, prostaglandin is administered to the bovine in addition to administration of the rFSH analog. The prostaglandin is optionally administered as a single dose, typically by injection, or as multiple doses administered several hours apart. In one embodiment, a first dose of prostaglandin is given to the bovine after administration of the rFSH analog followed by a second dose of prostaglandin which is given to the bovine approximately 6 hours to 1 day following the first prostaglandin dose.

The use according to a particular embodiment further comprises inseminating said bovine near the time of ovulation and, preferably, 2 to 7 days, more preferably 4 to 6 days after rFSH administration.

The use according to a particular embodiment further comprises collecting oocytes from said bovine, preferably near the time of ovulation and, more preferably, 2 to 7 days, even more preferably 4 to 6 days after rFSH analog administration.

Alternatively, the use of the invention comprises a step (c) of recovering or collecting oocytes from a treated bovine of (b), preferably near the time of ovulation and, more preferably, 2 to 7 days, even more preferably 4 to 6 days after rFSH analog administration. Oocytes may be recovered by techniques known per se in the art such as, without limitation, follicular aspiration. The collected oocytes display improved characteristics for fecundity. The collected oocytes may be fertilized *in vitro,* and subsequently transferred to recipient bovines.

The invention is used in any bovine. It is particularly suited for treating female beef and dairy cattle, including heifers.

Further aspects and advantages of the invention will be disclosed in the following experimental section, which illustrates the claimed invention.

### EXAMPLES

### EXAMPLE 1 - EFFECT ON REPRODUCTION PERFORMANCE OF A SINGLE 50µg rFSH ANALOG ADMINISTRATION IN SYNCHRONIZED NON-HUMAN MAMMALS

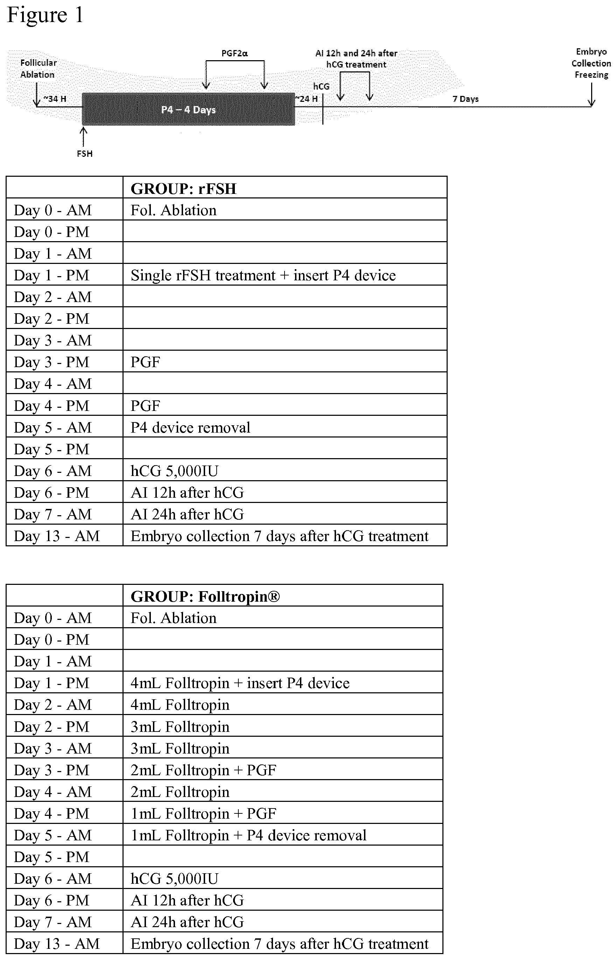

We treated 14 dairy heifers with one single intramuscular injection of bovine rFSH analog (50µg/heifer). Injection was performed 30h (in a 24 to 48 h window) after follicular ablation. The bovine rFSH analog used is as depicted in Figure 2. The treatment protocol is represented in Figure 1.

All groups received two injections of prostaglandin PGF2α at 2 days and 3 days after the first FSH injection (F0). hCG (human Chorionic Gonadotropin) injection was performed at 5 days after the F0 and Artificial Insemination (AI) was performed 12h and 24h after the hCG treatment. Embryo collection by non-surgical procedure occurred 7 days after AI.

Folltropin®-V (Bioniche Animal Health Product, Canada) was used a reference treatment. Folltropin®-V is a purified lyophilized follitropin extract obtained from porcine pituitary glands. Folltropin®-V (50 mg) was administered intramuscularly, twice daily, in decreasing doses during four days (cumulated FSH at the end of treatment is 400mg/heifer). 14 dairy heifers were treated with Folltropin®-V.

Different parameters were measured:
- Superovulation: superovulation was considered successful if > 2CL (Corpora Lutea) on the day of the flush;
- Ovulation rate: based on overall group LSM (Least Squares Means) of ovulatory response per cow;
- Embryo quality: based on overall group LSM (Least Squares Means) of number of viable embryos per cow;

The results are presented in the following table.

| | rFSH/single injection 50µg |
|---|---|
| Heifers enrolled (n) | 14 |
| Heifers superovulated (n)* | 13 |
| Heifers superovulated (%) | 92.8 |
| Ovulation rate | 81% |
| Average number of CL | 14.2 |
| Number of viable embryos (VE) | 7.9 |
| VE vs Folltropin®-V VE | 134% |

The results show that the treatment of the invention can clearly induce superovulation response in cattle. As a comparison, in the heifers treated with the reference protocol (8 injections of a natural FSH such as Folltropin®-V, 400mg), the treatment of the invention (1 single injection of a rFSH analog, 50µg) resulted in a higher ovulation rate (81% vs 76%) and a higher number of viable embryos (7.9 vs 5.9). Accordingly, even if one heifer did not superovulate with the treatment of the invention (while all did with the reference treatment), the quality of the embryos seem better with the invention. Accordingly, the combination of a follicle synchronization treatment with a single rFSH analog injection allows consistent production of superovulated embryos.

### EXAMPLE 2 - EFFECT ON REPRODUCTION PERFORMANCE OF A SINGLE 100µg rFSH ANALOG ADMINISTRATION IN SYNCHRONIZED NON-HUMAN MAMMALS (not according to the invention)

We treated 14 dairy heifers with one single intramuscular injection of bovine rFSH analog (100µg/heifer). Injection was performed 30h (in a 24 to 48 h window) after follicular ablation. The bovine rFSH analog used is as depicted in Figure 2. The treatment protocol is represented in Figure 1.

All groups received two injections of prostaglandin PGF2α at 2 days and 3 days after the first FSH injection (F0). hCG (human Chorionic Gonadotropin) injection was performed at 5 days after the F0 and Artificial Insemination (AI) was performed 12h and 24h after the hCG treatment. Embryo collection by non-surgical procedure occurred 7 days after AI.

Folltropin®-V (Bioniche Animal Health Product, Canada) was used a reference treatment. Folltropin®-V is a purified lyophilized follitropin extract obtained from porcine pituitary glands. Folltropin®-V (50 mg) was administered intramuscularly, twice daily, in decreasing doses during four days (cumulated FSH at the end of treatment is 400mg/heifer). 14 dairy heifers were treated with Folltropin®-V.

Different parameters were measured:
- Superovulation: superovulation was considered successful if > 2CL (Corpora Lutea) on the day of the flush;
- Ovulation rate: based on overall group LSM (Least Squares Means) of ovulatory response per cow;
- Embryo quality: based on overall group LSM (Least Squares Means) of number of viable embryos per cow;

The results are presented in the following table.

| | rFSH/single injection 100µg |
|---|---|
| Heifers enrolled (n) | 14 |
| Heifers superovulated (n)* | 12 |
| Heifers superovulated (%) | 85.7 |
| Ovulation rate | 88% |
| Average number of CL | 14.7 |
| Number of viable embryos (VE) | 3.8 |

The results show that the treatment of the invention can clearly induce superovulation response in cattle. As a comparison, in the heifers treated with the reference protocol (8 injections of a natural FSH such as Folltropin®-V, 400mg), the treatment of the invention (1 single injection of a rFSH analog, 100µg) resulted in a higher ovulation rate (88% vs 76%). Accordingly, even if two heifers did not superovulate with the treatment of the invention (while all did with the reference treatment), the quality of the embryos seem equivalent between the two products. The treatment achieved more than 70% of superovulatory response and also met the criteria in terms of embryo quality. Accordingly, the combination of a follicle synchronization treatment with a single rFSH analog injection allows consistent production of superovulated embryos.

## Claims

1. A use of a bovine recombinant FSH (rFSH) analog or a composition comprising a bovine recombinant rFSH analog to increase reproductive performance in a bovine, wherein said bovine
recombinant rFSH analog comprises an amino acid sequence represented by SEQ ID NO 3 and said rFSH analog is administered to said bovine after follicle synchronization, in one single administration at a dose of 50 µg.

2. The use according to claim 1, wherein said rFSH is administered by systemic administration, preferably by intramuscular administration, more preferably by injection.

3. The use according to any one of claims 1 or 2, wherein said rFSH has an amino acid sequence represented by SEQ ID NO 3.

4. The use according to any one of claims 1 to 3, further comprising inseminating said bovine near the time of ovulation and, preferably, 2 to 7 days, more preferably 4 to 6 days after rFSH administration.

5. The use according to any one of claims 1 to 4, further comprising collecting oocytes from said bovine, preferably near the time of ovulation and, more preferably, 2 to 7 days, even more preferably 4 to 6 days after rFSH analog administration.

6. The use according to any one of claims 1-5, comprising:
(a) providing a bovine or a group of bovines, which has been treated to synchronize follicles;
(b) administering to said bovine or group of bovines one single dose of said rFSH analog at a dose of 50 µg, preferably by injection, more preferably by intramuscular injection; and
(c) optionally inseminating the bovine or group of bovines, preferably by artificial insemination, or collecting oocytes from the bovine or group of bovines, preferably near the time of ovulation and, more preferably, 2 to 7 days, even more preferably 4 to 6 days after administration step (b).

7. The use according to any one of claims 1-5, comprising:
(a) treating a bovine or a group of bovines, to synchronize follicles;
(b) administering to said bovine or group of bovines one single dose of said rFSH analog at a dose of 50 µg, preferably by injection, more preferably by intramuscular injection; and
(c) optionally inseminating the bovine or group of bovines, preferably by artificial insemination, or collecting oocytes from the bovine or group of bovines, preferably near the time of ovulation and, more preferably, 2 to 7 days, even more preferably 4 to 6 days after administration step (b).

8. The use according to anyone of claims 1-7, wherein follicle synchronization is obtained by hormonal treatment of the bovine, preferably with PGF2alpha or its analogs, GnRH and PGF2alpha, or progestagen, optionally in combination with estrogen or PGF2alpha or GnRH, or by follicular ablation.

9. The use according to any one of claims 1 to 8, wherein the reproductive performance is increased by inducing superovulation in the bovine.

10. The use according to any one of claims 1 to 8, wherein the reproductive performance is increased by increasing pregnancy in the bovine.

11. The use according to any one of claims 1 to 8, wherein the reproductive performance is increased by increasing ovulation rate in said bovine.

12. The use according to any one of claims 1 to 11, wherein the bovine is a female beef or dairy cattle, including a heifer.

## Patentansprüche

1. Eine Verwendung eines bovinen rekombinanten FSH-(rFSH)-Analogons oder einer Zusammensetzung umfassend ein bovines rekombinantes rFSH-Analogon zur Erhöhung der Reproduktionsleistung in einem Rind, wobei besagtes bovines rekombinantes rFSH-Analogon eine Aminosäuresequenz umfasst, die von SEQ ID NR: 3 dargestellt ist, und besagtes rFSH-Analogon an besagtes Rind nach follikulärer Synchronisierung in einer einzigen Verabreichung mit einer Dosis von 50 µg verabreicht wird.

2. Die Verwendung gemäß Anspruch 1, wobei besagtes rFSH mittels systemischer Verabreichung, bevorzugt mittels intramuskulärer Verabreichung, bevorzugter mittels Injektion, verabreicht wird.

3. Die Verwendung gemäß einem der Ansprüche 1 oder 2, wobei besagtes rFSH eine von SEQ ID NR: 3 dargestellte Aminosäuresequenz besitzt.

4. Die Verwendung gemäß einem der Ansprüche 1 bis 3, außerdem umfassend Insemination besagten Rinds zeitnah zur Ovulation und bevorzugt 2 bis 7 Tage, bevorzugter 4 bis 6 Tage nach rFSH-Verabreichung.

5. Die Verwendung gemäß einem der Ansprüche 1 bis 4, außerdem umfassend Sammeln von Oocyten von besagtem Rind, bevorzugt zeitnah zur Ovulation und bevorzugter 2 bis 7 Tage, noch bevorzugter 4 bis 6 Tage nach rFSH-Analogon-Verabreichung.

6. Die Verwendung gemäß einem der Ansprüche 1 bis 5, umfassend:
(a) Bereitstellen eines Rinds oder eine Gruppe von Rindern, die behandelt worden sind, um Follikel zu synchronisieren;
(b) Verabreichen an besagtes Rind oder besagte Gruppe von Rindern einer Einzeldosis von besagtem rFSH-Analogon mit einer Dosis von 50 µg, bevorzugt mittels Injektion, bevorzugter mittels intramuskulärer Injektion; und
(c) gegebenenfalls Insemination des Rinds oder der Gruppe von Rindern, bevorzugt mittels künstlicher Insemination oder Sammeln von Oocyten von dem Rind oder der Gruppe von Rindern, bevorzugt zeitnah zur Ovulation und bevorzugter 2 bis 7 Tage, noch bevorzugter 4 bis 6 Tage nach Verabreichungsschritt (b).

7. Die Verwendung gemäß einem der Ansprüche 1 bis 5, umfassend:
(a) Behandeln eines Rinds oder einer Gruppe von Rindern, um Follikel zu synchronisieren;
(b) Verabreichen an besagtes Rind oder besagte Gruppe von Rindern einer Einzeldosis von besagtem rFSH-Analogon mit einer Dosis von 50 µg, bevorzugt mittels Injektion, bevorzugter mittels intramuskulärer Injektion; und
(c) gegebenenfalls Insemination des Rinds oder der Gruppe von Rindern, bevorzugt mittels künstlicher Insemination oder Sammeln von Oocyten von dem Rind oder der Gruppe von Rindern, bevorzugt zeitnah zur Ovulation und bevorzugter 2 bis 7 Tage, noch bevorzugter 4 bis 6 Tage nach Verabreichungsschritt (b).

8. Die Verwendung gemäß einem der Ansprüche 1 bis 7, wobei eine follikuläre Synchronisierung durch eine Hormonbehandlung des Rinds, bevorzugt mit PGF2alpha oder seinen Analoga, GnRH und PGF2alpha oder Progestagen, gegebenenfalls in Kombination mit Östrogen oder PGF2alpha oder GnRH, oder durch Follikelablation erhalten wird.

9. Die Verwendung gemäß einem der Ansprüche 1 bis 8, wobei die Reproduktionsleistung durch Induzieren einer Superovulation in dem Rind erhöht wird.

10. Die Verwendung gemäß einem der Ansprüche 1 bis 8, wobei die Reproduktionsleistung durch Erhöhung der Trächtigkeit in dem Rind erhöht wird.

11. Die Verwendung gemäß einem der Ansprüche 1 bis 8, wobei die Reproduktionsleistung durch Erhöhung der Ovulationsrate in besagtem Rind erhöht wird.

12. Die Verwendung gemäß einem der Ansprüche 1 bis 11, wobei das Rind ein weibliches Rind oder Milchkuh, einschließlich einer Färse, ist.

## Revendications

1. Utilisation d'un analogue de FSH recombinée (rFSH) bovine ou d'une composition comprenant un analogue de rFSH recombinée bovine pour accroître la performance reproductive chez un bovin, dans laquelle ledit analogue de rFSH recombinée bovine comprend une séquence d'acides aminés représentée par SEQ ID No 3 et ledit analogue de rFSH est administré audit bovin après synchronisation des follicules, en une seule administration à une dose de 50 µg.

2. Utilisation selon la revendication 1, dans laquelle ladite rFSH est administrée par voie systémique, de préférence par voie intramusculaire, plus préférablement par injection.

3. Utilisation selon l'une quelconque des revendications 1 ou 2, dans laquelle ladite rFSH a la séquence d'acides aminés représentée par SEQ ID No 3.

4. Utilisation selon l'une quelconque des revendications 1 à 3, comprenant en outre l'insémination dudit bovin à proximité de la période d'ovulation et, de préférence, 2 à 7 jours, plus préférablement 4 à 6 jours après l'administration de la rFSH.

5. Utilisation selon l'une quelconque des revendications 1 à 4, comprenant en outre le recueil des ovocytes dudit bovin, de préférence à proximité de la période d'ovulation et, plus préférablement, 2 à 7 jours, plus préférablement encore 4 à 6 jours après l'administration de l'analogue de rFSH.

6. Utilisation selon l'une quelconque des revendications 1 à 5, comprenant :
(a) l'utilisation d'un bovin ou groupe de bovins, qui a été traité pour synchroniser les follicules ;
(b) l'administration audit bovin ou groupe de bovins d'une dose unique dudit analogue de rFSH à une dose de 50 µg, de préférence par injection, plus préférablement par injection intramusculaire ; et
(c) éventuellement l'insémination du bovin ou groupe de bovins, de préférence par insémination artificielle, ou le recueil des ovocytes du bovin ou groupe de bovins, de préférence à proximité de la période d'ovulation et, plus préférablement, 2 à 7 jours, plus préférablement encore 4 à 6 jours après l'étape d'administration (b).

7. Utilisation selon l'une quelconque des revendications 1 à 5, comprenant :
(a) le traitement d'un bovin ou groupe de bovins, pour synchroniser les follicules ;
(b) l'administration audit bovin ou groupe de bovins d'une dose unique dudit analogue de rFSH à une dose de 50 µg, de préférence par injection, plus préférablement par injection intramusculaire ; et
(c) éventuellement l'insémination du bovin ou groupe de bovins, de préférence par insémination artificielle, ou le recueil des ovocytes du bovin ou groupe de bovins, de préférence à proximité de la période d'ovulation et, plus préférablement, 2 à 7 jours, plus préférablement encore 4 à 6 jours après l'étape d'administration (b).

8. Utilisation selon l'une quelconque des revendications 1 à 7, dans laquelle la synchronisation des follicules est obtenue par traitement hormonal du bovin, de préférence par PGF2alpha ou ses analogues, GnRH et PGF2alpha, ou progestagène, éventuellement en association avec un oestrogène ou PGF2alpha ou GnRH, ou par ablation folliculaire.

9. Utilisation selon l'une quelconque des revendications 1 à 8, dans laquelle la performance reproductive est accrue par induction d'une superovulation chez le bovin.

10. Utilisation selon l'une quelconque des revendications 1 à 8, dans laquelle la performance reproductive est accrue par accroissement de la gestation chez le bovin.

11. Utilisation selon l'une quelconque des revendications 1 à 8, dans laquelle la performance reproductive est accrue par accroissement du taux d'ovulation chez ledit bovin.

12. Utilisation selon l'une quelconque des revendications 1 à 11, dans laquelle le bovin est un boeuf femelle ou une vache laitière, y compris une génisse.


### SEQUENCE LISTING

<110> CEVA SANTE ANIMALE
<120> COMPOSITIONS AND METHODS FOR INCREASING REPRODUCTION PERFORMANCE IN NON HUMAN MAMMALS USING A BIOLOGICALLY ACTIVE RECOMBINANT FOLLICLE STIMULATING HORMONE (rFSH)
<130> B1406PC
<160> 4
<170> PatentIn version 3.3
<210> 1
   <211> 109
   <212> PRT
   <213> Bovine FSH alpha subunit
<400> 1
   Cys Glu Leu Thr Asn Ile Thr Ile Thr Val Glu Lys Glu Glu Cys Gly
   1               5                   10                  15
   Phe Cys Ile Ser Ile Asn Thr Thr Trp Cys Ala Gly Tyr Cys Tyr Thr
               20                  25                  30
   Arg Asp Leu Val Tyr Arg Asp Pro Ala Arg Pro Asn Ile Gln Lys Thr
           35                  40                  45
   Cys Thr Phe Lys Glu Leu Val Tyr Glu Thr Val Lys Val Pro Gly Cys
       50                  55                  60
   Ala His His Ala Asp Ser Leu Tyr Thr Tyr Pro Val Ala Thr Glu Cys
   65                  70                  75                  80
   His Cys Ser Lys Cys Asp Ser Asp Ser Thr Asp Cys Thr Val Arg Gly
                   85                  90                  95
   Leu Gly Pro Ser Tyr Cys Ser Phe Arg Glu Ile Lys Glu
               100                 105
<210> 2
   <211> 96
   <212> PRT
   <213> Bovine FSH beta subunit
<400> 2
   Phe Pro Asp Gly Glu Phe Thr Met Gln Gly Cys Pro Glu Cys Lys Leu
   1               5                   10                  15
   Lys Glu Asn Lys Tyr Phe Ser Lys Pro Asp Ala Pro Ile Tyr Gln Cys
               20                  25                  30
   Met Gly Cys Cys Phe Ser Arg Ala Tyr Pro Thr Pro Ala Arg Ser Lys
           35                  40                  45
   Lys Thr Met Leu Val Pro Lys Asn Ile Thr Ser Glu Ala Thr Cys Cys
       50                  55                  60
   Val Ala Lys Ala Phe Thr Lys Ala Thr Val Met Gly Asn Val Arg Val
   65                  70                  75                  80
   Glu Asn His Thr Glu Cys His Cys Ser Thr Cys Tyr Tyr His Lys Ser
                   85                  90                  95
<210> 3
   <211> 253
   <212> PRT
   <213> Bovine rFSH analog
<400> 3
   Met Lys Ser Val Gln Phe Cys Phe Leu Phe Cys Cys Trp Arg Ala Ile
   1               5                   10                  15
   Cys Cys Arg Ser Cys Glu Leu Thr Asn Ile Thr Ile Thr Val Glu Lys
               20                  25                  30
   Glu Glu Cys Gly Phe Cys Ile Ser Ile Asn Thr Thr Trp Cys Ala Gly
           35                  40                  45
   Tyr Cys Tyr Thr Arg Asp Leu Val Tyr Arg Asp Pro Ala Arg Pro Asn
       50                  55                  60
   Ile Gln Lys Thr Cys Thr Phe Lys Glu Leu Val Tyr Glu Thr Val Lys
   65                  70                  75                  80
   Val Pro Gly Cys Ala His His Ala Asp Ser Leu Tyr Thr Tyr Pro Val
                   85                  90                  95
   Ala Thr Glu Cys His Cys Ser Lys Cys Asp Ser Asp Ser Thr Asp Cys
               100                 105                 110
   Thr Val Arg Gly Leu Gly Pro Ser Tyr Cys Ser Phe Arg Glu Ile Lys
           115                 120                 125
   Glu Ser Ser Ser Ser Lys Ala Pro Pro Pro Ser Leu Pro Ser Pro Ser
       130                 135                 140
   Arg Leu Pro Gly Pro Ser Asp Thr Pro Ile Leu Pro Gln Phe Pro Asp
   145                 150                 155                 160
   Gly Glu Phe Thr Met Gln Gly Cys Pro Glu Cys Lys Leu Lys Glu Asn
   165 170 175
   Lys Tyr Phe Ser Lys Pro Asp Ala Pro Ile Tyr Gln Cys Met Gly Cys
   180 185 190
   Cys Phe Ser Arg Ala Tyr Pro Thr Pro Ala Arg Ser Lys Lys Thr Met
   195 200 205
   Leu Val Pro Lys Asn Ile Thr Ser Glu Ala Thr Cys Cys Val Ala Lys
   210 215 220
   Ala Phe Thr Lys Ala Thr Val Met Gly Asn Val Arg Val Glu Asn His
   225 230 235 240
   Thr Glu Cys His Cys Ser Thr Cys Tyr Tyr His Lys Ser
   245 250
<210> 4
   <211> 762
   <212> DNA
   <213> Bovine rFSH analog
<400> 4
   atgaagtctg tccagttctg tttccttttc tgttgctgga gagcaatctg ctgcagaagc 60
   tgcgagctga ccaacatcac catcacggtg gagaaagagg aatgtggctt ctgcataagc 120
   atcaacacca cgtggtgtgc aggctactgc tacacccggg acttggtgta cagggaccca 180
   gcaaggccca atatccagaa aacgtgtacc ttcaaggagc tggtctacga gacggtgaaa 240
   gtgcctggct gtgctcacca tgcagactcc ctgtacacgt acccagtagc cactgaatgt 300
   cactgcagca agtgcgacag cgacagcact gactgcaccg tgagaggcct ggggcccagc 360
   tactgctcct tcagggaaat caaagaatcc tcttcctcaa aggcccctcc cccgagcctt 420
   ccaagtccat cccgactccc ggggccctcg gacaccccga tcctcccaca atttcctgat 480
   ggagagttta caatgcaggg ctgtcctgaa tgcaagctaa aagaaaacaa atacttctcc 540
   aagccagatg ctccaatcta tcagtgcatg gggtgctgct tctccagggc ataccccact 600
   ccagcgaggt ctaagaagac aatgttggtc cccaagaaca tcacctcgga agctacatgc 660
   tgtgtggcca aagcatttac caaggccaca gtgatgggaa atgtcagagt ggagaaccac 720
   accgagtgcc actgcagcac ttgttattat cacaaatcct aa 762